# EUROPEAN PATENT APPLICATION

(11) **EP 0 727 486 A2**
(43) Date of publication of application: **21.08.1996**
(21) Application number: 95101980.1
(22) Date of filing: 14.02.1995
(51) Int. Cl.: C12N 15/12, C07K 14/47, C12N 5/10, C12P 21/08, C12Q 1/68

(54) **Tumour suppressor gene**

(71) Applicant: CANCER INSTITUTE, Toshima-ku, Tokyo (JP); Eisai Co., Ltd., Tokyo 112 (JP)
(72) Inventor: Nakamura, Yusuke, Yokohama-shi, Kanagawa (JP); Imai, Takashi, Narashino-shi, Chiba (JP)
(74) Representative: Hansen, Bernd, Dr. Dipl.-Chem.

(57) **Abstract**

A detailed genetic map on human chromosome 11 was prepared. Then, a commonly deleted region on the chromosome in the tumor tissues of patients with multiple endocrine neoplasia type 1 was identified. Further, by the linkage analysis on a family line with this disease, a gene causative of this disease was localized. A gene present in the region common to these observations was cloned and the structure of this gene was determined. Because a protein coded by this DNA is homologous with those of transcriptional factors, it is expected that the above-mentioned gene may be a novel tumor suppressor gene. Further, it is also expected that the above-mentioned gene and a protein coded for thereby may be useful in preparations of a remedy for cancer and a diagnostic drug for cancer.

## Description

### Background of the Invention

### Field of the Invention

The present invention relates to a human tumor suppressor gene, a polypeptide coded for thereby and a gene analysis method wherein the above-mentioned gene is used. Thus, they are usable in the field of medicines.

### Description of the Related Art

It has been known for a long time that gene mutation in cells plays an important role in the onset of cancer. Recent advances in genetic engineering have made it possible to amplify specific DNAs and to analyze gene mutation in cancer cells and thus contributed to the remarkable development in the field of studies on cancer.

Analysis and identification of oncogenes, which are thought to participate in the cancerization of cells and the abnormal proliferation of cancer cells, are now in progress and the number of the oncogenes thus clarified so far amounts to several tens. On the other hand, tumor suppressor genes having a reverse function have been the focus of intense research interest in these several years. Examples of the tumor suppressor genes which have been found out so far include Rb gene of retinoblastoma [Friend, S.H., et al., Proc. Natl. Acad. Sci. USA., 84, 9095 (1987)], p53 gene [Lane, D.P., et al., Nature, 278, 261 (1979)] and APC gene [Kenneth, W.K., et al., Science, 253, 661 (1991)] of colon cancer and WT1 gene of Wilms' tumor [Call, K.M., et al., Cell, 60, 509 (1990)]. In the case of the p53 gene, it is known that this mutation gene has been handed down over generations as a germ-line in ceratin family lines ["Li-Fraumeni syndrome"; Makin, D., et al., Science, 250, 1233 (1990); and Srivastava, S., et al., Nature, 348, 747 (1990)]. However, it is considered that there are much more unidentified tumor suppressor genes.

Multiple endocrine neoplasia type 1 (MEN1) is an autosomal dominant hereditary disease characterized by the development of hyperplasia or neoplasm in the endocrine organs such as accessory thyroid, islets of Langerhans in the pancreas and pituitary gland [Brandi, M.L., et. al., Endocr. Rev. 8, 391 (1987)]. It is assumed by linkage studies that a genetic defect exists in the long arm of chromosome 11 (11q). Also there is known a region which is deleted with high frequency on chromosome 11q in MEN 1-associated tumors. Based on these facts, it is considered that a tumor suppressor gene exists in this region.

Accordingly, it is now the focus of world-wide interest of physicians and researches to isolate this tumor suppressor gene, to clarify its role in the disease and to clarify its biological function. Thus it has been urgently required to isolate the tumor suppressor gene in this region.

It is an object of the present invention to provide a novel tumor suppressor gene, a transformant transformed by a plasmid having, integrated therein, the full structure or part of the tumor suppressor gene, a polypeptide which is coded for by the tumor suppressor gene, an antibody against the polypeptide and methods for studying, examining, diagnosing and medically treating cancer with the use of them.

### Disclosure of the Invention

### Summary of the Invention

The present inventors isolated cosmid clones containing a number of RFLP markers on chromosome 11 and prepared a detailed genetic map. By using these newly developped RFLP markers, a region deleted commonly in such tumors was further localized. And, a region where the target tumor suppressor gene existed was restricted to through the linkage analysis. As a result, the region common to these observations was specified as 11q13. From among cosmid clones of this region, those containing exons were selected. By using a fragment thereof as a probe, a cDNA library was screened. Thus a cDNA coding for an amino acid sequence being homologous with transcriptional factors such as human Wilms' tumor suppressor gene (WT1) product and human early growth response protein 2 (EGR2) was isolated.

An organism specifically responds to various exogenous and endogenous stimuli by comprehensively utilizing, for example, its nervous, immune, circulatory and endocrine systems. After being input, information is transmitted via the so-called information transmitting system or enters directly into nuclei and thus acts on a gene or a transcriptional factor. As a result, the expression of the gene is modified and thus cells begin to take a turn for the differentiation, proliferation (cancerization) or death. From the very beginning, the process of the ontogeny and morphogenesis of an organism or the sustenance of its life per se is merely the results of the cascade mechanism of gene expression. Thus, it is not too much to say that nothing but "the coordination in gene expression depending mainly on transcription" makes a living organism as it is and cancer breaks out when this coordination falls into disorder.

Therefore, we deemed the clone thus isolated as one of tumor suppressor genes, isolated the cDNA thereof in the full length and analyzed the structure thereof. As a result, it has been proved that a protein which is coded for by this cDNA in the full length is an intranuclear transcriptional regulator having a nuclear localizing signal, a proline-rich domain and a zinc finger motif.

Thus, the present invention relates to:
(1) a DNA comprising the full structure or a part of the DNA represented by SEQ ID NO:1;
(2) a polypeptide comprising the full structure or a part of the polypeptide coded for by the DNA represented by SEQ ID NO:1;
(3) a transformant transformed by a plasmid having, integrated therein, the full structure or a part of the DNA represented by SEQ ID NO:1 which can be expressed therein;
(4) an antibody against the above-mentioned polypeptide as an antigen; and
(5) a gene analysis method which comprises using, as a primer, a probe or a marker, a DNA comprising a part of the DNA represented by SEQ ID NO:1 and hybridizing the primer, the probe or the marker with a DNA to be tested.

In other words, the present invention relates to:
(a) a cDNA which comprises one containing the full or a part of the cDNA of the tumor suppressor gene represented by SEQ ID NO:1;
(b) a polypeptide which comprises one containing the full or a part of the polypeptide coded for by the cDNA of the tumor suppressor gene represented by SEQ ID NO:1;
(c) host cells which are obtained by integrating the full or a part of the cDNA described in SEQ ID NO:1 into a plasmid which can express it and transforming thereby;
(d) an antibody against the polypeptide described in the above item (b) as an antigen; and
(e) a gene analysis method characterized by using a DNA containing a part of the DNA sequence described in the above item (a) as a primer, a probe or a marker.

With respect to the DNAs and polypeptides, those which are substantially equivalent to the DNAs and polypeptides described above are also included in the scope of the present invention. The expression "DNAs and polypeptides being substantially equivalent" means those which have been modified via, for example, deletion, replacement, addition or insertion of the constituting bases or constituting amino acids and derivatives thereof, which exhibit the same effects as those of the original DNAs or polypeptides. However, the extent of these effects is irrelevant thereto. The term "a part of the DNA" means a fragment composed of at least 10 bases derived from the DNA. In order to employ as a primer, for example, a DNA fragment having a base sequence generally consisting of 10 to 30 bases, preferably 15 to 25 bases, is selected. In order to employ as a probe, a DNA fragment having a base sequence generally consisting of at least 15 bases, preferably at least 20 bases, is selected.

The term "a part of the polypeptide" means a peptide having a sequence composed of at least 6 amino acid residues derived from the polypeptide. When a part of a polypeptide is to be used as an antigen for the preparation of an antibody or as an epitope for the detection of an antibody, it is known that a peptide having a sequence consisting of 6 amino acid residues would bind to an antibody [see WO 8403564, published on Sep. 13, 1984 (Assignee: COMMONWEALTH SERUM LABS and GEYSEN, H. M.)]. A peptide having a sequence generally consisting of at least 10 amino acid residues, preferably at least 20 amino acid residues, is employed therefor. Although it may be anticipated that a peptide having a sequence consisting of 6 amino acid residues can achieve only a poor efficiency in the production of an antibody, such a peptide is also usable in the form of a fused peptide.

Furthermore, an RNA which comprises one translated from the DNA represented by SEQ ID NO:1 or a part of the same and RNAs which are substantially equivalent thereto are included in the scope of the present invention.

Now the present invention will be described in greater detail.

### Detailed Description of the Invention

### (1) Isolation of cDNA

The target cosmid library of the human chromosome 11 can be prepared in, for example, the following manner. From human/mouse hybrid cell line containing a single human chromosome 11 in a mouse genomic background, a chromosomal DNA is extracted. Then DNA fragments of the chromosomal DNA can be integrated into a vector such as pWE15 by a conventional method [Maniatis, T., et al., Molecular Cloning 2nd. ed., Cold Spring Harbor Laboratory Press, N.Y. (1989)]. Clones having an insert originating in the human chromosome can be screened by the colony hybridization with the use of a whole human DNA as probe. The thus obtained cosmid clones containing a DNA originating in the human chromosome 11 are then subjected to the fluorescent in-situ hybridization (FISH) method [Takahashi et al., Am. J. Hum. Genet., 86, 14 - 16 (1990)]. Thus, each of the multitude of the cosmid clones can be localized throughout the chromosome and a physical chromosomal map can be prepared. Further, RFLP markers can be isolated on the basis of the fragment length pattern which has been prepared by cleaving human DNA with several restriction enzymes [Nakamura et al., Am. J. Hum. Genet., 43, 854 - 859 (1988)]. Among these clones, those located around the region of 11q13 are subjected to the FISH method and the linkage analysis to thereby give a further detailed genetic map. Based on this map, the DNA of a cancer tissue of a patient is examined in the loss of heterozygosity (LOH) by utilizing the RFLP. Thus the region where the target tumor suppressor gene is located can be further restricted to.

From the cosmid clones existing in the region which has been thus restricted to, a DNA fragment being under expression can be isolated by the exon trapping method [Buckler, A., et al., Proc. Natl. Acad. Sci. USA, 88, 4005 - 4009 (1991)]. By using the DNA fragment thus obtained as probe, the cDNA of a gene existing in the restricted region near q13 of human chromosome 11 can be cloned.

### (2) Confirmation of the whole structure of the gene

The base sequence of the cDNA can be determined by the Maxam-Gilbert method [Maxam, A.M. and Gilbert, W., Proc. Natl. Acad. Sci. USA, 74, 560 (1977)] or the dideoxy technique [Messing, J., Nucleic acid Res., 9, 309 (1981)].

It can be confirmed by, for example, the 5'RACE method, the 3'RACE method or the Northern blotting that the cDNA obtained by the above-mentioned method contains the full length protein translation region.

### (3) Recombinant expression vectors and transformants transformed thereby

The tumor suppressor gene cDNA obtained by the above-mentioned method, or a fragment thereof is integrated into an appropriate vector and then this vector is introduced into appropriate host cells to obtain a transformant. By culturing this transformant in a conventional manner, a large amount of the tumor suppressor gene product, or a fragment thereof can be obtained from the culture. More specifically, the cDNA is linked to the downstream side of the promoter of a vector suitable for the expression of the cDNA by a known method with the use of restriction enzymes and DNA ligase. Thus a recombinant expression vector can be constructed. Examples of the vectors usable therefor include plasmids pRB322 and pUC18 originating in Escherichia coli, plasmid pUB110 originating in Bacillus subtilis, plasmid pRB15 originating in yeast, phage vectors λgt10 and λgt11, and vector SV40 originating in animal virus, though any vector capable of replicating and amplifying in the host cells may be used therefor without restriction. Similarly the promoter and the terminator are not restricted in particular and any suitable combination may be selected therefor depending on the host to be used, so long as they are adapted for the host employed in the expression of a DNA sequence coding for the tumor suppressor gene, or a fragment thereof. Any DNA may be used as the cDNA herein so long as it codes for the tumor suppressor gene product, or a fragment thereof. A chemically synthesized one may be used therefor. When the protein to be expressed is one having a physiological activity of suppressing the proliferation of cancer cells, then the sequence of the cDNA is not restricted to the DNA sequence represented by the SEQ ID NO:1 but a DNA having a DNA sequence which has undergone partial substitution, deletion, insertion or a combination thereof may be used therefor as the cDNA.

The recombinant expression vector thus obtained is introduced into a host by, for example, the competent cell method [J. Mol. Biol., 53, 154 (1970)], the protoplast method [Proc. Natl. Acad. Sci. USA, 75, 1929 (1978)], the calcium phosphate method [Science, 221, 551 (1983)], the in vitro packaging method [Proc. Natl. Acad. Sci. USA, 72, 581 (1975)] or the virus vector method [Cell, 37, 1053 (1984)] to thereby prepare a transformant. Escherichia coli, Bacillus subtilis, yeasts and animal cells are usable as the host. The transformant thus obtained is then cultured in an appropriate medium selected depending on the employed host. The culture is usually effected at a temperature of from 20 to 45°C within a pH range of from 5 to 8 and, if necessary, under aeration and/or stirring. The tumor suppressor gene product or a fragment thereof may be separated and purified from the culture by appropriately combining known separation/isolation methods. Examples of these methods include salting out, solvent precipitation, dialysis, gel filtration, electrophoresis, ion exchange chromatography, affinity chromatography and reversed phase high performance liquid chromatography.

### (4) Preparation of antibody

By using the tumor suppressor gene product or a fragment thereof as an antigen, an antibody is prepared. A polyclonal antibody is prepared in accordance with a conventional method by, for example, sufficiently immunizing an animal such as mouse, guinea pig and rabbit with the antigen by subcutaneously, intramuscularly, intraperitoneally or intravenously administering it a number of times, sampling the blood from the animal and then separating the serum to obtain the antibody. A commercially available adjuvant is also usable therefor.

A monoclonal antibody can be prepared by a known method. For example, spleen cells of a mouse immunized with the antigen described above are fused with commercially available mouse myeloma cells to thereby give hybridomas. Then the target monoclonal antibody can be prepared from the culture supernatant of the hybridoma or the ascites fluid of a mouse inoculated with the hybridoma.

It is not necessary that the tumor suppressor gene product to be used as the antigen has the whole amino acid structure but a peptide having a partial structure thereof, a modified peptide, its derivative or a fused peptide formed by fusing this peptide with another peptide are also usable. These substances may be prepared by any of the biological technique and chemical synthesis technique.

These antibodies enable the identification and determination of the peptide of the present invention in human biological samples and thus are applicable to, for example, diagnostic drugs for diseases to which the polypeptide is related. The peptide can be immunologically assayed in accordance with any of the known methods including the fluorescent antibody method, the passive agglutination method and the enzyme-labeled antibody technique.

### (5) Gene analysis of human organic tissues

Examples of the biological sample usable in the gene analysis include normal human tissues, various types of human tumor tissues, human blood, human bodily fluids and human secretions. The DNA of the employed tissue may be extracted and prepared by, for example, the method reported by Sato, T., et al. [Cancer Res., 50, 7184 (1990)].

From the DNA sequence provided by the present invention, a part DNA sequence at an appropriate position is selected and a synthetic oligonucleotide having this sequence or one complementary thereto is used as a primer, a probe or a marker. Thus the occurrence of a mutation of this gene in man and the morphology of the mutation can be analyzed. Furthermore, alterations (insertion, deletion, etc.) of this gene in a sample can also be detected by these analyses.

The part DNA sequence may be selected from any part of the DNA sequence of the above-mentioned gene. It is needless to say that an artificially modified DNA sequence may be used therefor and thus the corresponding gene mutation can be detected.

The analysis may be effected by, for example, the following method. Namely, primers of two sequences are selected and the partial sequence between them is amplified by the PCR method. Then the amplified DNA sequence is directly analyzed. Alternatively, this amplification product is integrated into a plasmid in the same manner as that of the above-mentioned case and host cells are transformed thereby. After culturing the transformant thus obtained, the DNA sequence of the clone thus obtained is analyzed. Further, the ligase chain reaction method may be applied to the amplification [Wu et al., Genomics, 4, 560 - 569 (1989)]. Furthermore, a specific mutation in the above-mentioned gene in a sample can be detected by using the allele-specific PCR [Ruano and Kidd, Nucleic Acid Research, 17, 8392 (1989)] or the ARMS method [C.R. Newton et al., Nucleic Acid Research, 17, 2503 - 2517 (1989)].

Similarly, a point mutation can be detected by the SSCP method [Orita et al., Proc. Natl. Acad. Sic. USA, 86, 2766 - 2770 (1989); and Genomics, 5, 874 - 879 (1989)] or the RNase-protection method with the use of probes containing the DNA sequence thus selected or an RNA sequence originating therein. By using these probes, a mutation in the above-mentioned gene in a sample can be detected by the Southern hybridization method or an abnormality in the expression level of this gene in a sample can be examined by the Northern hybridization method.

Escherichia coli DH5αF'/pAB1, pFL2 and pCE9 each carrying a plasmid containing the cDNA of this tumor suppressor gene were deposited with National Institute of Bioscience and Human-Technology, Agency of Industrial Science and Technology, Ministry of International Trade and Industry under accession numbers FERM P-14127, 14128 and 14129, respectively, on February 8, 1994, and they were changed to International deposition under accession numbers FERM BP-4923, 4924 and 4925, respectively, on December 9, 1994.

The DNA of the present invention has a structure homologous with those of transcriptional factors, and originates in the most restricted commonly deleted region on chromosome 11 in MEN 1-associated tumors. Therefore, it is expected that the DNA of the present invention may be a novel tumor suppressor gene. The DNA may be used as a tool in a gene therapy. Further, the fragment of the DNA may be used in the gene analysis of the DNA and in the diagnosis of diseases to which the DNA relates.

The polypeptide coded for by the DNA according to the present invention may be used as a reagent for investigations and used for preparing an antibody. The antibody may be used in the qualitative or quantitative analysis of the polypeptide in a biological sample. Thus, it is expected that the antibody may be useful as a novel diagnostic drug.

### Brief Description of the Drawings

Fig. 1 is a diagram showing the restriction of the region in which the MEN1 gene exists by the linkage analysis and the LOH analysis.

Fig. 2 is a diagram showing cDNA clones which overlap one another and the domain structure of ZFM1 cDNA derived therefrom.

Fig. 3 is a diagram showing the homology of the ZFM1 protein with WT1 or EGR2.

Fig. 4 is a diagram showing the constitution of exons of the ZFM1 gene. The exons are represented by 1 to 14. The domains observed in cDNA are represented by A to H.

### Examples

To further illustrate the present invention in greater detail and in particular, the following Examples will be given. However it is to be understood that the present invention is not restricted these Examples only.

### Example 1 Isolation and linkage analysis of cosmid clones specific for chromosome 11

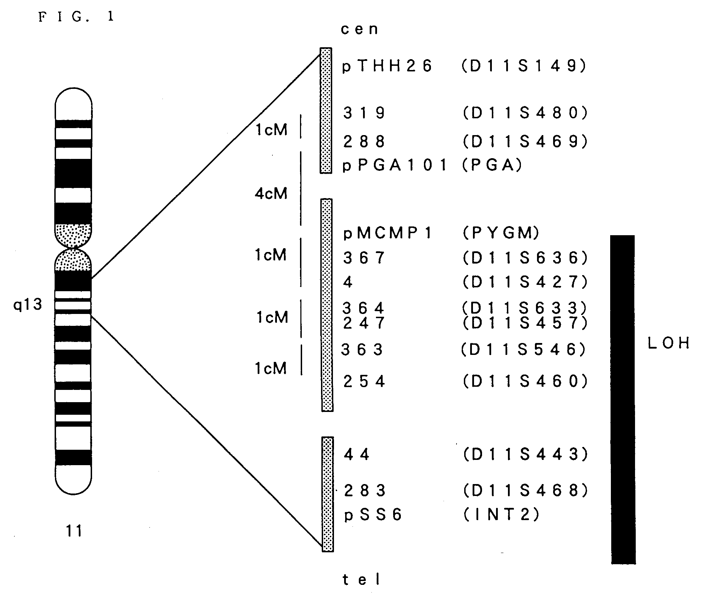

At the early stage of studies, it was reported based on the linkage with a PYGM (muscle glycogen phosphorylase gene) marker that a gene participating in the onset of MENI existed in the long arm of chromosome 11 [Larrson et al., Nature, 332, 85 - 87 (1988)]. Subsequently, it was reported that it existed in a region of 12cM located between D11S149 maker and INT2 marker of 11q13 [Nakamura et al., Am. J. Hum. Genet., 44, 751 - 755 (1989)]. We prepared a cosmid library from a Chinese hamster/human hybrid cell line containing a single human chromosome 11 and screened cosmid clones containing a part of the human chromosomal DNA with the use of a whole human DNA as probe [Tokino et al., Am. J. Hum. Genet., 48, 258 - 268 (1991); and Tanigami et al., Am. J. Hum. Genet., 50, 56 - 64 (1992)]. Then, these clones were tested by hybridization with a hybrid cell line panel containing a part of human chromosome 11 [Tanigami et al., Am. J. Hum. Genet., 50, 56 - 64 (1992)] and were mapped on the chromosome through the fluorescent in-situ hybridization (FISH) method [Hori et al., Genomics, 13, 129 - 133 (1992)]. By effecting the linkage analysis with the use of the cosmid markers whereby RFLP could be detected, the location of the MEN1 gene was restricted to a region of 8cM between D11S480 (cCI11-319) and D11S546 (cCI11-363) on q13 of chromosome 11 [Fujimori et al., Am. J. Hum. Genet., 50, 399 - 403 (1992)] (see Fig. 1).

### Example 2 Preparation of deletion map of chromosome 11 in MEN1-associated tumors

On the other hand, investigations on the loss of heterozygosity (LOH) of the chromosome 11 in MEN1-associated tumors have also suggested that the tumor suppressor gene exists in the above-mentioned region [Friedman et al., N. Engl. J. Med., 321, 213 - 218 (1989); Thakker et al., N. Engl. J. Med., 321, 218 - 224 (1989); and Bale et al., Cancer Res., 51, 1154 - 1157 (1991)]. It has been further pointed out by the mapping of the deleted region on chromosome 11q in these tumors that the MEN1 gene exists in the telomere side of PYGM [Bystroen et al., Proc. Nat. Acad. Sci. USA, 87, 1968 - 1972 (1990)]. The results of the examination on LOH are arranged together with the results of the linkage analysis and it is thus considered that the MEN1 gene exists in a region of about 3cM between PYGM and D11S546 (see Fig. 1).

### Example 3 Preparation of physical map of 11q13 region

We cleaved human genomic DNA with 8 restriction enzymes each having a rare breakage point. After separating DNA fragments by the pulse field gel electrophoresis, the Southern blotting analysis was carried out by using more than 50 cosmid clones existing in 11q13 as probes. Thus, the relationship in locations among the cosmid clones has been clarified depending upon the capability of each clone of being hybridized with a common genomic DNA fragment. As a result, it has been found out that cCI11-4, cCI11-367, cCI11-364, cCI11-247, cCI11-363, cCI11-254 and PYGM can be hybridized with genomic DNA fragments relating to one another and thus they are located within a range of about 1 Mb in the telomere side of PYGM [Tanigami et al., Genomics, 13, 21 - 24 (1992)]. It has been suggested that PYGM and cCI11-4, among these cosmid clones, are markers closest to the MEN1 gene (lod values: 5.03 and 5.13) [Fujimori et al., Am. J. Hum. Genet., 50, 399 - 403 (1992)]. Based on the results of the mapping of the breakage points with restriction enzymes in YAC clones 1908F2 and 199A7 isolated by using PYGM as a probe, it has been clarified that cCI11-367, among the 6 cosmid clones as described above, is also close to PYGM.

### Example 4 Isolation of exon sequence from 11q13 region

As described above, cCI11-4 and cCI11-367 are cosmid clones which are closest to the MEN1 gene. Thus, an attempt was made to isolate exons from these 2 cosmid clones by the exon trapping method [Buckler, A., et al., Proc. Natl. Acad. Sci. USA, 88, 4005 - 4009 (1991)]. The cosmid DNA was cleaved with BglII or BamHI, or both of these enzymes, and the fragment thus obtained was linked to the BamHI site of an exon splicing vector pSPL1. Transfection into COS-7 cells and isolation of exon sequences by the reverse transcription PCR (RT-PCR) were effected each in accordance with the procedure described in the original paper. Consequently, 3 exon sequences originating in cCI11-367 were obtained and named respectively s367EI, s367E2 and s367E4. These exon sequences were respectively in sizes of 147 bp, 76 bp and 129 bp.

### Example 5 Isolation of full-length cDNA

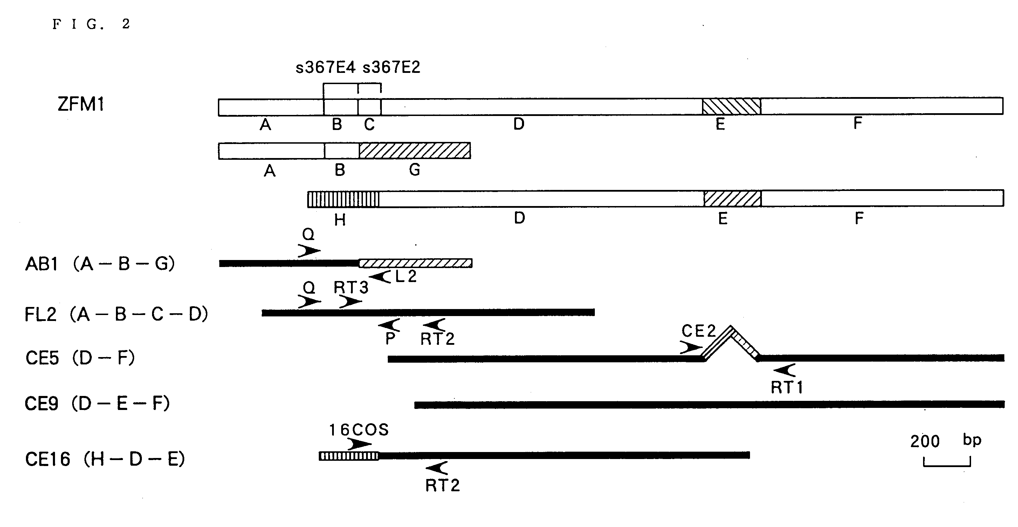

By using s367E4 (i.e., one of the exon sequences obtained in the above Example 4) as probe, a human cortical cDNA library was screened. Thus, a clone AB1 carrying a cDNA insert of 1 kb was obtained. With the use of this clone AB1 as a probe, further, a cDNA clone FL2 was obtained from a human fetal liver cDNA library while cDNA clones CE5, CE9 and CE16 were obtained from a human cerebellar cDNA library. Then, it was confirmed that each of these clones could be hybridized with the original cosmid clone cCI11-367 and mapped on the chromosome 11q13 with a hybrid cell line panel. A sequence constructed by overlapping these cDNA clones one another at the common parts corresponded to ZFM1 cDNA of 3200 bp (SEQ ID NO:1). This ZFM1 cDNA contained an open reading frame (ORF) of 1869 bp which corresponded to a sequence of base Nos. 383 to 2251 in SEQ ID NO:1. Based on the information as will be described hereinbelow, it has been proved that the sequence of SEQ ID NO: 1 and that of each clone can be regarded as being composed of 6 domains A (base Nos. 1 to 413 in SEQ ID NO:1), B (base Nos. 414 to 542 in SEQ ID NO:1), C (base Nos. 543 to 618 in SEQ ID NO:1), D (base Nos. 619 to 1964 in SEQ ID NO:1), E (base Nos. 1965 to 2218 in SEQ ID NO:1) and F (base Nos. 2219 to 3200 in SEQ ID NO:1) and domains G and H which are completely different therefrom. Namely, the exon sequences s367E2 and s367E4 obtained in the above Example 4 corresponded respectively to the domains C and B. The cDNA clone CE5 lacked in a domain E consisting of 254 base pairs corresponding to a sequence of base Nos. 1965 to 2218 in SEQ ID NO:1, which may be due to an alternative splicing. The cDNA clone AB1 contained domains A and B and the different one G but not the domains C, D, E and F. The cDNA clone CE16 consisted of the domains D and E and the different one H (see Fig. 2).

### Example 6 Characteristics of the structure of protein coded for by the tumor suppressor gene

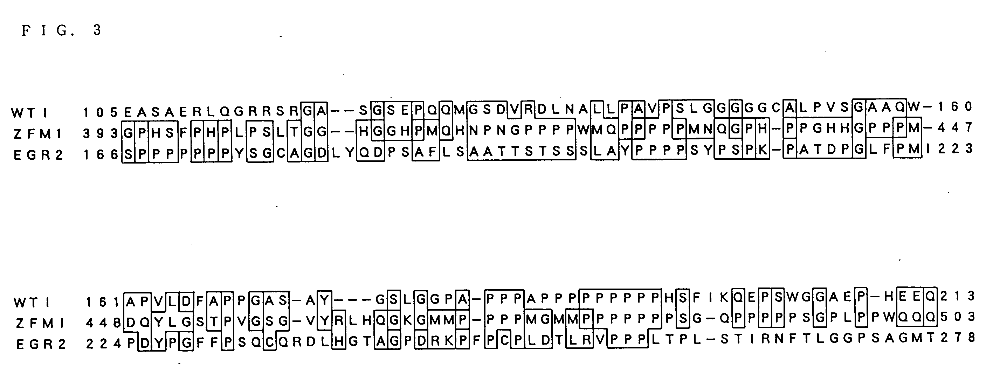

A protein coded for by ZFM1 cDNA consisted of 623 amino acid residues and had a nuclear localizing signal containing basic amino acids in the N-terminal side. Further, a sequence C-X2-C-X4-H-X4-C (amino acid Nos. 279 - 292) had characteristics of a zinc finger motif existing in a DNA binding protein. 118 proline residues were contained in this ZFM1 protein. In particular, 69 proline residues were contained in a region of amino acid Nos. 420 to 623 thereof. The sequence of this region showed high homologies with Wilms' tumor suppressor gene product (WT1) [Gessler et al., Nature, 343, 774 - 778 (1990)] and early growth response 2 (EGR2) protein as a transcriptional factor (27.3% and 24.0%, respectively) (see Fig. 3). WT1 is a transcription factor having a Kruppel-like zinc finger motif [Rosenberg et al., Nature, 319, 336 - 339 (1986)]. EGR2 is a human homologue of an early growth response gene Krox-20 [Chavier et al., EMBO J. 7, 29 - 35 (1988)] which is expressed at the G0-G1 junction in the cell cycle of cultured mouse cells and it is also a transcriptional factor [Joseph et al., Proc. Natl. Acad. Sci. USA, 85, 7164 - 7168 (1988)]. The ZFM1 protein further had 7 proline repetitive sequences (each consisting of at least 4 proline residues located continuously) in the C-terminal side. One of these repetitive sequences followed a glutamine repetitive sequence and thus formed a structure which was almost the same as that of the hinge domain of a mineralocorticoid receptor [Arriza et al., Science, 237, 268 - 275 (1987)]. Such a hinge structure is essentially required in the communication between a hormone binding domain and a DNA binding domain [Krust et al., EMBO J., 5, 891 - 897 (1986); and Giguere et al., Cell, 46, 645 - 652 (1986)]. Further, mRNAs of a number of types originating in the ZFM1 gene were expressed in hormone-producing organs such as pancreas, thyroid, adrenal gland and ovarium (see Table 1 in Example 8).

These facts indicate that the ZFM1 protein is a tumor suppressor gene which is localized in the nuclei and exerts its function by binding to DNA and thus suppressing the proliferation of cells and that ZFM1 is a gene which participates in the onset of MEN1.

### Example 7 Structure of genomic gene

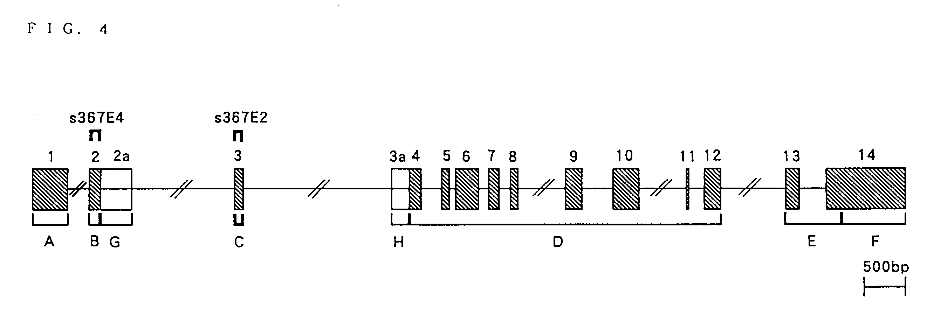

Based on the cosmid clone containing the ZFM1 gene, the genomic structure of the ZFM1 gene was determined. The ZFM1 gene existed over a region of about 20 kb in the genomic DNA and consisted of 14 exons (see Fig. 4). As Fig. 4 shows, it has been revealed that these exons (Nos. 1 to 14) and the domains A to H described in the above Example 6 relate to each other as follows: domain A = exon 1, domain B = exon 2, domain C = exon 3, domain D = exon 4, 5, 6, 7, 8, 9, 10, 11 and 12, domain E = exon 13 and a part of exon 14, domain F = a part of exon 14, domain G = exon 2a, and domain H = exon 3a.

The sequence of SEQ ID NO: 1 contains all of these 14 exons except the exons 2a and 3a. The sequence of the cDNA clone CE5 consisting of the domains D-F lacks in the domain E corresponding to the exon 13 and a part of the exon 14. On the other hand, the domain G of the cDNA clone AB1 consisting of the domains A-B-G is coded for by the exon 2a which directly follows the exon 2 coding for the domain B. Also, the domain H of the cDNA clone CE16 consisting of the domains H-D-E is coded for by the exon 3a which is located immediately before the exon 4 coding for the domain D.

### Example 8 Expression of ZFM1 gene in human tissues

By using an insert of the cDNA clone FL2 as a probe, mRNAs of various tissues were analyzed by the Northern blotting method. As a result, the expressions of ZFM1 mRNAs of 3.3 kb and 2.7 kb were observed in all of these tissues. It is considered that the larger mRNA corresponds to the full length cDNA containing the domains A-B-C-D-E-F, while the smaller mRNA corresponds to one containing the domain H instead of the domains A-B-C (see Fig. 2). To examine the expression of the ZFM1 gene in greater detail, the reverse transcription PCR (RT-PCR) analysis was effected by extracting RNAs from various human tissues and using primer sets (see the arrow heads in Fig. 2) specific for the respective domains. As a result, the expressions of ZFM1 mRNAs of various types, which were thought to be due to differences in splicing, were observed over a wide range of tissues. The expressions of 3 mRNAs having structures of A-B-C-D, A-B-G and H-D (see Fig. 2) were observed in nearly all tissues, though the expression yields differed from one another. In contrast, the expression of a mRNA having the domain E was restricted to heart, pancreas, thyroid and ovarium (see Table 1).

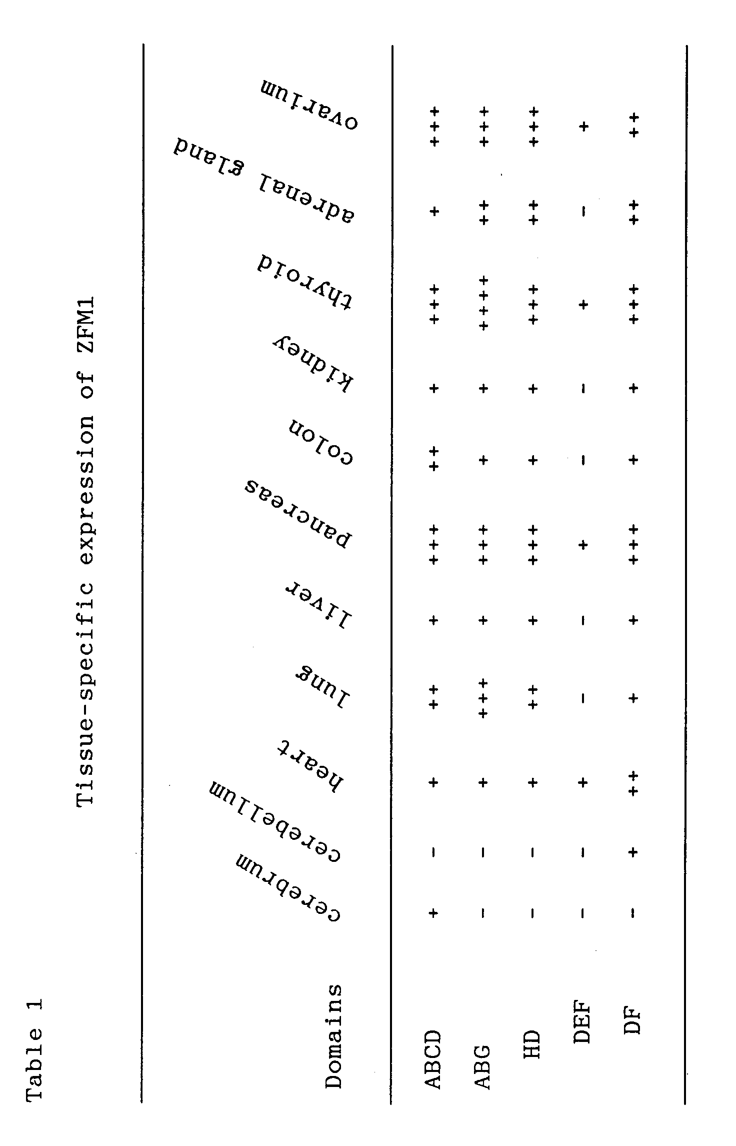

SEQUENCE LISTING
(2) INFORMATION FOR SEQ ID NO: 1:
(i) SEQUENCE CHARACTERISTICS:
(A) LENGTH: 3200 base pairs
(B) TYPE: nucleic acid
(C) STRANDEDNESS: double
(D) TOPOLOGY: linear
(ii) MOLECULE TYPE: cDNA
(vi) ORIGINAL SOURCE:
(A) ORGANISM: Homo sapiens
(ix) FEATURE:
(A) FEATURE KEY: 5'UTR
(B) LOCATION: 1..382
(A) FEATURE KEY: CDS
(B) LOCATION: 383..2254
(A) FEATURE KEY: exon 1
(B) LOCATION: 1..413
(A) FEATURE KEY: exon 2
(B) LOCATION: 414..542
(A) FEATURE KEY: exon 3
(B) LOCATION: 543..618
(A) FEATURE KEY: exon 4
(B) LOCATION: 619..771
(A) FEATURE KEY: exon 5
(B) LOCATION: 772..861

(A) FEATURE KEY: exon 6
(B) LOCATION: 862..1045
(A) FEATURE KEY: exon 7
(B) LOCATION: 1046..1161
(A) FEATURE KEY: exon 8
(B) LOCATION: 1162..1269
(A) FEATURE KEY: exon 9
(B) LOCATION: 1270..1450
(A) FEATURE KEY: exon 10
(B) LOCATION: 1451..1724
(A) FEATURE KEY: exon 11
(B) LOCATION: 1725..1784
(A) FEATURE KEY: exon 12
(B) LOCATION: 1785..1964
(A) FEATURE KEY: exon 13
(B) LOCATION: 1965..2137
(A) FEATURE KEY: exon 14
(B) LOCATION: 2138..3132
(A) FEATURE KEY: 3'UTR
(B) LOCATION: 2280..3200
(xi) SEQUENCE DESCRIPTION: SEQ ID NO:1:
CGTTGCTGTC GAAATGAAGT GCGCGCTGCG ACACCTCCCA GCCCACCGAA CTCCGCCGCC 60
ATTTCCTCGC TTGCCTAACG GTTCGGCCAA TCCCAGCGCG CATCAATGCC GGACTGAGGC 120
TCCGCCAATC GGAGGCCGCC GATTTCGACC CTTCGCCTCG GCCCGGCCCA ATCCATTCCC 180
CGGCCCCGCC GCCCCCGGCC CGCCCCCGCG GTGCCCTCTC TCCTCCCTCT TTGTGCGTCT 240

CGCGCCGCCG CCGCCCGCCG CGTGAGAGGA CGGGCTCCGC GCGCTCCGGC AGCGCATTCG 300

GGTCCCCTCC CCCCGGGAGG CTTGCGAAGG AGAAGCCGCC GCAGAGGAAA AGCAGGTGCC 360

GGTGCCTGTC CCCGGGGGCG CC ATG GCG ACC GGA GCG AAC GCC ACG CCG TTG 412

Met Ala Thr Gly Ala Asn Ala Thr Pro Leu

1               5                   10

GAC TTC CCA AGT AAG AAG CGG AAG AGG AGC CGC TGG AAC CAA GAC ACA 460

Asp Phe Pro Ser Lys Lys Arg Lys Arg Ser Arg Trp Asn Gln Asp Thr

                15                  20                  25

ATG GAA CAG CCG ACA GTG ATT CCA GGA ATG CCT ACA GTT ATT CCC CCT 508

Met Glu Gln Pro Thr Val Ile Pro Gly Met Pro Thr Val Ile Pro Pro

            30                  35                  40

GGA CTT ACT CGA GAA CAA GAA AGA GCT TAT ATA GTG CAA CTG CAG ATA 556

Gly Leu Thr Arg Glu Gln Glu Arg Ala Tyr Ile Val Gln Leu Gln Ile

        45                  50                  55

GAA GAC CTG ACT CGT AAA CTG CGC ACA GGG GAC CTG GGC ATC CCC CCT 604

Glu Asp Leu Thr Arg Lys Leu Arg Thr Gly Asp Leu Gly Ile Pro Pro

    60                  65                  70

AAC CCT GAG GAC AGG TCC CCT TCC CCT GAG CCC ATC TAC AAT AGC GAG 652

Asn Pro Glu Asp Arg Ser Pro Ser Pro Glu Pro Ile Tyr Asn Ser Glu

75                  80                  85                  90

GGG AAG CGG CTT AAC ACC CGA GAG TTC CGC ACC CGC AAA AAG CTG GAA 700

Gly Lys Arg Leu Asn Thr Arg Glu Phe Arg Thr Arg Lys Lys Leu Glu

                95                  100                 105

GAG GAG CGG CAC AAC CTC ATC ACA GAG ATG GTT GCA CTC AAT CCG GAT 748
Glu Glu Arg His Asn Leu Ile Thr Glu Met Val Ala Leu Asn Pro Asp
            110                 115                 120
TTC AAG CCA CCT GCA GAT TAC AAA CCT CCA GCA ACA CGT GTG AGT GAT 796
Phe Lys Pro Pro Ala Asp Tyr Lys Pro Pro Ala Thr Arg Val Ser Asp
        125                 130                 135
AAA GTC ATG ATT CCA CAA GAT GAG TAC CCA GAA ATC AAC TTT GTG GGG 844
Lys Val Met Ile Pro Gln Asp Glu Tyr Pro Glu Ile Asn Phe Val Gly
    140                 145                 150
CTG CTC ATC GGG CCC AGA GGG AAC ACC CTG AAG AAC ATA GAG AAG GAG 892
Leu Leu Ile Gly Pro Arg Gly Asn Thr Leu Lys Asn Ile Glu Lys Glu
155                 160                 165                 170
TGC AAT GCC AAG ATT ATG ATC CGG GGG AAA GGG TCT GTG AAA GAA GGG 940
Cys Asn Ala Lys Ile Met Ile Arg Gly Lys Gly Ser Val Lys Glu Gly
                175                 180                 185
AAG GTT GGG CGC AAA GAT GGC CAG ATG TTG CCA GGA GAA GAT GAG CCA 988
Lys Val Gly Arg Lys Asp Gly Gln Met Leu Pro Gly Glu Asp Glu Pro
            190                 195                 200
CTT CAT GCC CTG GTT ACT GCC AAT ACA ATG GAG AAC GTC AAA AAG GCA 1036
Leu His Ala Leu Val Thr Ala Asn Thr Met Glu Asn Val Lys Lys Ala
        205                 210                 215
GTG GAA CAG ATA AGA AAC ATC CTG AAG CAG GGT ATC GAG ACT CCA GAG 1084
Val Glu Gln Ile Arg Asn Ile Leu Lys Gln Gly Ile Glu Thr Pro Glu
    220                 225                 230

GAC CAG AAT GAT CTA CGG AAG ATG CAG CTT CGG GAG TTG GCT CGC TTA 1132
Asp Gln Asn Asp Leu Arg Lys Met Gln Leu Arg Glu Leu Ala Arg Leu
235                 240                 245                 250
AAT GGG ACC CTT CGG GAA GAC GAT AAC AGG ATC TTA AGA CCC TGG CAG 1180
Asn Gly Thr Leu Arg Glu Asp Asp Asn Arg Ile Leu Arg Pro Trp Gln
                255                 260                 265
AGC TCA GGG ACC CGC AGC ATT ACC AAC ACC ACA GTG TGT ACC AAG TGT 1228
Ser Ser Gly Thr Arg Ser Ile Thr Asn Thr Thr Val Cys Thr Lys Cys
            270                 275                 280
GGA GGG GCT GGC CAC ATT GCT TCA GAC TGT AAA TTC CAA AGG CCT GGT 1276
Gly Gly Ala Gly His Ile Ala Ser Asp Cys Lys Phe Gln Arg Pro Gly
        285                 290                 295
GAT CCT CAG TCA GCT CAG GAT AAA GCA CGG ATG GAT AAA GAA TAT TTG 1324
Asp Pro Gln Ser Ala Gln Asp Lys Ala Arg Met Asp Lys Glu Tyr Leu
    300                 305                 310
TCC CTC ATG GCT GAA CTG GGT GAA GCA CCT GTC CCA GCA TCT GTG GGC 1372
Ser Leu Met Ala Glu Leu Gly Glu Ala Pro Val Pro Ala Ser Val Gly
315                 320                 325                 330
TCC ACC TCT GGG CCT GCC ACC ACA CCC CTG GCC AGC GCA CCT CGT CCT 1420
Ser Thr Ser Gly Pro Ala Thr Thr Pro Leu Ala Ser Ala Pro Arg Pro
                335                 340                 345
GCT GCT CCC GCC AAC AAC CCA CCT CCA CCG TCT CTC ATG TCT ACC ACC 1468
Ala Ala Pro Ala Asn Asn Pro Pro Pro Pro Ser Leu Met Ser Thr Thr
            350                 355                 360

CAG AGC CGC CCA CCC TGG ATG AAT TCT GGT CCT TCA GAG AGT TGG CCC 1516
Gln Ser Arg Pro Pro Trp Met Asn Ser Gly Pro Ser Glu Ser Trp Pro
        365                 370                 375
TAC CAC GGC ATG CAT GGA GGT GGT CCT GGT GGG CCC GGA GGT GGC CCC 1564
Tyr His Gly Met His Gly Gly Gly Pro Gly Gly Pro Gly Gly Gly Pro
    380                 385                 390
CAC AGC TTC CCA CAC CCA TTA CCC AGC CTG ACA GGT GGG CAT GGT GGA 1612
His Ser Phe Pro His Pro Leu Pro Ser Leu Thr Gly Gly His Gly Gly
395                 400                 405                 410
CAT CCC ATG CAG CAC AAC CCC AAT GGA CCC CCA CCC CCT TGG ATG CAG 1660
His Pro Met Gln His Asn Pro Asn Gly Pro Pro Pro Pro Trp Met Gln
                415                 420                 425
CCA CCA CCA CCA CCG ATG AAC CAG GGC CCC CAC CCT CCT GGG CAC CAT 1708
Pro Pro Pro Pro Pro Met Asn Gln Gly Pro His Pro Pro Gly His His
            430                 435                 440
GGC CCT CCT CCA ATG GAT CAG TAC CTG GGA AGT ACG CCT GTG GGC TCT 1756
Gly Pro Pro Pro Met Asp Gln Tyr Leu Gly Ser Thr Pro Val Gly Ser
        445                 450                 455
GGG GTC TAT CGC CTG CAT CAA GGA AAA GGT ATG ATG CCG CCA CCA CCT 1804
Gly Val Tyr Arg Leu His Gln Gly Lys Gly Met Met Pro Pro Pro Pro
    460                 465                 470
ATG GGC ATG ATG CCG CCG CCG CCG CCG CCT CCC AGT GGG CAG CCC CCA 1852
Met Gly Met Met Pro Pro Pro Pro Pro Pro Pro Ser Gly Gln Pro Pro
475                 480                 485                 490

CCC CCT CCC TCT GGT CCT CTT CCC CCA TGG CAA CAA CAG CAG CAG CAG 1900

Pro Pro Pro Ser Gly Pro Leu Pro Pro Trp Gln Gln Gln Gln Gln Gln

                495                 500                 505

CCT CCG CCA CCC CCT CCG CCC AGC AGC AGT ATG GCT TCC AGT ACC CCC 1948

Pro Pro Pro Pro Pro Pro Pro Ser Ser Ser Met Ala Ser Ser Thr Pro

            510                 515                 520

TTG CCA TGG CAG CAA AAT ACG ACG ACT ACC ACC ACG AGC GCT GGC ACA 1996

Leu Pro Trp Gln Gln Asn Thr Thr Thr Thr Thr Thr Ser Ala Gly Thr

        525                 530                 535

GGG TCC ATC CCG CCA TGG CAA CAG CAG CAG GCG GCT GCC GCA GCT TCT 2044

Gly Ser Ile Pro Pro Trp Gln Gln Gln Gln Ala Ala Ala Ala Ala Ser

    540                 545                 550

CCA GGA GCC CCT CAG ATG CAA GGC AAC CCC ACT ATG GTG CCC CTG CCC 2092

Pro Gly Ala Pro Gln Met Gln Gly Asn Pro Thr Met Val Pro Leu Pro

555                 560                 565                 570

CCC GGG GTC CAG CCG CCT CTG CCG CCT GGG GCC CCT CCC CCT CCG CCC 2140

Pro Gly Val Gln Pro Pro Leu Pro Pro Gly Ala Pro Pro Pro Pro Pro

                575                 580                 585

CGT AGC ATC GAG TGT CTT CTT TGT CTT CTT TCT CTC CTC ACC CAA CTC 2188

Arg Ser Ile Glu Cys Leu Leu Cys Leu Leu Ser Leu Leu Thr Gln Leu

            590                 595                 600

CCT TTG CCT CTC CCC AAA CCG GGC CGC CAG GAT CCC TCC CCG CGG CGG 2236

Pro Leu Pro Leu Pro Lys Pro Gly Arg Gln Asp Pro Ser Pro Arg Arg

        605                 610                 615

CGA TGG CCC GAG CCA TGAGAGTGAG GACTTTCCGC GCCCATTGGT GACCCTTCCA 2291

Arg Trp Pro Glu Pro

620 623

GGCAGACAGC CTCAGCAACG CCCCTGGTGG ACAGGATGGT TCGGCAAAGC AGCCTGAGTT 2351

ATTTTTGTGG ACGGAATCGG AACACGCTGG CTCCATATCG TGAAATTTTT ATTAATTTTT 2411

TTCTTTTTCC TTTGTTACTT CTTTATCTTT TCCTTTCTTC AGACTCCGTC CAAGGAGATG 2471

CTCTCCCCGG TCTTCTGCTG CAATTTAGAT TCCTTTGGGT TCTCTCCAGT TCTCCTTCCC 2531

TTACCAAGGA GAGGGGAGCA AATGGTTTTG GGCAAGGGCT TTGGCCATTC ATGTCAAGCT 2591

GGTTGTGGGT TTTTCAAGGT GCCATAGCCA CCCCCAAATA TGTTTGTTTA AAGCGTGGGG 2651

TTTTTTAATC TCTGCCACCC TTGTCAAGGG AGTCTTGTAA AGTTGCCGAG GGTAGGTTCA 2711

TCTCCAGGTT TCGGGATTCC CATCCGTCCT GGCGATCCTG CCAGCAGTGG GTGGGCAGCC 2771

TGAGCTCCCT CGGGCTCGCC TGCCAGCCTG GAGTTCTTCC TGTGCTCCTT GATCACCTGA 2831

GCTGCCTCAG ATTCCATTTG GTCCTCTCCT TCCTGGAAGG CTTCCTTTTA TGTTTTGTTT 2891

TAATCCCAAA TGTCTGAATG TTTTGCAGTG TGTAGGGGTT TGAGCCCCTT GTTCATTCTC 2951

CTTCCTTTTT CCTCCCGCTT CCCTCTCCAT GAAGTGATTC TGTTGACAAT AATGTATACT 3011

GCGCGTTCTC TTCACTGGTT,TATCTGCAGA,AATTTCTCTG GGCTTTTTTC GGTGTTAGAT 3071

TCAACACTGC GCTAAAGCGG,GGATGTTCCA,TTGAATAAAA GAGCAGTGTG GTTTTCTGGG 3131

AAAAAAAAAA AAAAAAAAAA,AAAAAAAAAA,AAAAAAAAAA AAAAAAAAAA AAAAAAAAAA 3191

AAAAAAAAA 3200

SEQUENCE LISTING

(1) GENERAL INFORMATION:

(i) APPLICANT:
(A) NAME: Cancer Institute
(B) STREET: 37-1, Kamiikebukuro 1-chome,
(C) CITY: Toshima-ku, Tokyo
(E) COUNTRY: Japan
(F) POSTAL CODE (ZIP): none

(A) NAME: Eisai Co., Ltd.
(B) STREET: 6-10, Koishikawa 4-chome,
(C) CITY: Bunkyo-ku, Tokyo
(E) COUNTRY: JAPAN
(F) POSTAL CODE (ZIP): 112

(ii) TITLE OF INVENTION: Tumor Suppressor Gene

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 95101980.1

(2) INFORMATION FOR SEQ ID NO: 1:

(i) SEQUENCE CHARACTERISTICS:
(A) LENGTH: 3200 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: 5'UTR
(B) LOCATION:1..382

(ix) FEATURE:
(A) NAME/KEY: CDS
(B) LOCATION:383..2254

(ix) FEATURE:
(A) NAME/KEY: exon 1
(B) LOCATION:1..413

(ix) FEATURE:
(A) NAME/KEY: exon 2
(B) LOCATION:414..542

(ix) FEATURE:
(A) NAME/KEY: exon 3
(B) LOCATION:543..618

(ix) FEATURE:
(A) NAME/KEY: exon 4
(B) LOCATION:619..771
(ix) FEATURE:
(A) NAME/KEY: exon 5
(B) LOCATION:772..861

(ix) FEATURE:
(A) NAME/KEY: exon 6
(B) LOCATION:862..1045

(ix) FEATURE:
(A) NAME/KEY: exon 7
(B) LOCATION:1046..1161

(ix) FEATURE:
(A) NAME/KEY: exon 8
(B) LOCATION:1162..1269

(ix) FEATURE:
(A) NAME/KEY: exon 9
(B) LOCATION:1270..1450

(ix) FEATURE:
(A) NAME/KEY: exon 10
(B) LOCATION:1451..1724

(ix) FEATURE:
(A) NAME/KEY: exon 11
(B) LOCATION:1725..1784

(ix) FEATURE:
(A) NAME/KEY: exon 12
(B) LOCATION:1785..1964

(ix) FEATURE:
(A) NAME/KEY: exon 13
(B) LOCATION:1965..2137

(ix) FEATURE:
(A) NAME/KEY: exon 14
(B) LOCATION:2138..3132

(ix) FEATURE:
(A) NAME/KEY: 3'UTR
(B) LOCATION:2280..3200

(xi) SEQUENCE DESCRIPTION: SEQ ID NO: 1:

CGTTGCTGTC GAAATGAAGT GCGCGCTGCG ACACCTCCCA GCCCACCGAA CTCCGCCGCC 60

ATTTCCTCGC TTGCCTAACG GTTCGGCCAA TCCCAGCGCG CATCAATGCC GGACTGAGGC 120

TCCGCCAATC GGAGGCCGCC GATTTCGACC CTTCGCCTCG GCCCGGCCCA ATCCATTCCC 180

CGGCCCCGCC GCCCCCGGCC CGCCCCCGCG GTGCCCTCTC TCCTCCCTCT TTGTGCGTCT 240

CGCGCCGCCG CCGCCCGCCG CGTGAGAGGA CGGGCTCCGC GCGCTCCGGC AGCGCATTCG 300

GGTCCCCTCC CCCCGGGAGG CTTGCGAAGG AGAAGCCGCC GCAGAGGAAA AGCAGGTGCC 360

GGTGCCTGTC CCCGGGGGCG CC ATG GCG ACC GGA GCG AAC GCC ACG CCG TTG 412
Met Ala Thr Gly Ala Asn Ala Thr Pro Leu
1               5                   10

GAC TTC CCA AGT AAG AAG CGG AAG AGG AGC CGC TGG AAC CAA GAC ACA 460
Asp Phe Pro Ser Lys Lys Arg Lys Arg Ser Arg Trp Asn Gln Asp Thr
                15                  20                  25

ATG GAA CAG CCG ACA GTG ATT CCA GGA ATG CCT ACA GTT ATT CCC CCT 508
Met Glu Gln Pro Thr Val Ile Pro Gly Met Pro Thr Val Ile Pro Pro
            30                  35                  40

GGA CTT ACT CGA GAA CAA GAA AGA GCT TAT ATA GTG CAA CTG CAG ATA 556
Gly Leu Thr Arg Glu Gln Glu Arg Ala Tyr Ile Val Gln Leu Gln Ile
        45                  50                  55

GAA GAC CTG ACT CGT AAA CTG CGC ACA GGG GAC CTG GGC ATC CCC CCT 604
Glu Asp Leu Thr Arg Lys Leu Arg Thr Gly Asp Leu Gly Ile Pro Pro
    60                  65                  70

AAC CCT GAG GAC AGG TCC CCT TCC CCT GAG CCC ATC TAC AAT AGC GAG 652
Asn Pro Glu Asp Arg Ser Pro Ser Pro Glu Pro Ile Tyr Asn Ser Glu
75                  80                  85                  90

GGG AAG CGG CTT AAC ACC CGA GAG TTC CGC ACC CGC AAA AAG CTG GAA 700
Gly Lys Arg Leu Asn Thr Arg Glu Phe Arg Thr Arg Lys Lys Leu Glu
                95                  100                 105

GAG GAG CGG CAC AAC CTC ATC ACA GAG ATG GTT GCA CTC AAT CCG GAT 748
Glu Glu Arg His Asn Leu Ile Thr Glu Met Val Ala Leu Asn Pro Asp
            110                 115                 120

TTC AAG CCA CCT GCA GAT TAC AAA CCT CCA GCA ACA CGT GTG AGT GAT 796
Phe Lys Pro Pro Ala Asp Tyr Lys Pro Pro Ala Thr Arg Val Ser Asp
        125                 130                 135

AAA GTC ATG ATT CCA CAA GAT GAG TAC CCA GAA ATC AAC TTT GTG GGG 844
Lys Val Met Ile Pro Gln Asp Glu Tyr Pro Glu Ile Asn Phe Val Gly
    140                 145                 150

CTG CTC ATC GGG CCC AGA GGG AAC ACC CTG AAG AAC ATA GAG AAG GAG 892
Leu Leu Ile Gly Pro Arg Gly Asn Thr Leu Lys Asn Ile Glu Lys Glu
155                 160                 165                 170

TGC AAT GCC AAG ATT ATG ATC CGG GGG AAA GGG TCT GTG AAA GAA GGG 940
Cys Asn Ala Lys Ile Met Ile Arg Gly Lys Gly Ser Val Lys Glu Gly
                175                 180                 185

AAG GTT GGG CGC AAA GAT GGC CAG ATG TTG CCA GGA GAA GAT GAG CCA 988
Lys Val Gly Arg Lys Asp Gly Gln Met Leu Pro Gly Glu Asp Glu Pro
            190                 195                 200

CTT CAT GCC CTG GTT ACT GCC AAT ACA ATG GAG AAC GTC AAA AAG GCA 1036
Leu His Ala Leu Val Thr Ala Asn Thr Met Glu Asn Val Lys Lys Ala
        205                 210                 215

GTG GAA CAG ATA AGA AAC ATC CTG AAG CAG GGT ATC GAG ACT CCA GAG 1084
Val Glu Gln Ile Arg Asn Ile Leu Lys Gln Gly Ile Glu Thr Pro Glu
    220                 225                 230

GAC CAG AAT GAT CTA CGG AAG ATG CAG CTT CGG GAG TTG GCT CGC TTA 1132
Asp Gln Asn Asp Leu Arg Lys Met Gln Leu Arg Glu Leu Ala Arg Leu
235                 240                 245                 250

AAT GGG ACC CTT CGG GAA GAC GAT AAC AGG ATC TTA AGA CCC TGG CAG 1180
Asn Gly Thr Leu Arg Glu Asp Asp Asn Arg Ile Leu Arg Pro Trp Gln
                255                 260                 265

AGC TCA GGG ACC CGC AGC ATT ACC AAC ACC ACA GTG TGT ACC AAG TGT 1228
Ser Ser Gly Thr Arg Ser Ile Thr Asn Thr Thr Val Cys Thr Lys Cys
            270                 275                 280

GGA GGG GCT GGC CAC ATT GCT TCA GAC TGT AAA TTC CAA AGG CCT GGT 1276

Gly Gly Ala Gly His Ile Ala Ser Asp Cys Lys Phe Gln Arg Pro Gly
        285                 290                 295

GAT CCT CAG TCA GCT CAG GAT AAA GCA CGG ATG GAT AAA GAA TAT TTG        1324
Asp Pro Gln Ser Ala Gln Asp Lys Ala Arg Met Asp Lys Glu Tyr Leu
    300                 305                 310

TCC CTC ATG GCT GAA CTG GGT GAA GCA CCT GTC CCA GCA TCT GTG GGC        1372
Ser Leu Met Ala Glu Leu Gly Glu Ala Pro Val Pro Ala Ser Val Gly
315                 320                 325                 330

TCC ACC TCT GGG CCT GCC ACC ACA CCC CTG GCC AGC GCA CCT CGT CCT        1420
Ser Thr Ser Gly Pro Ala Thr Thr Pro Leu Ala Ser Ala Pro Arg Pro
                335                 340                 345

GCT GCT CCC GCC AAC AAC CCA CCT CCA CCG TCT CTC ATG TCT ACC ACC        1468
Ala Ala Pro Ala Asn Asn Pro Pro Pro Pro Ser Leu Met Ser Thr Thr
            350                 355                 360

CAG AGC CGC CCA CCC TGG ATG AAT TCT GGT CCT TCA GAG AGT TGG CCC        1516
Gln Ser Arg Pro Pro Trp Met Asn Ser Gly Pro Ser Glu Ser Trp Pro
        365                 370                 375

TAC CAC GGC ATG CAT GGA GGT GGT CCT GGT GGG CCC GGA GGT GGC CCC        1564
Tyr His Gly Met His Gly Gly Gly Pro Gly Gly Pro Gly Gly Gly Pro
    380                 385                 390

CAC AGC TTC CCA CAC CCA TTA CCC AGC CTG ACA GGT GGG CAT GGT GGA        1612
His Ser Phe Pro His Pro Leu Pro Ser Leu Thr Gly Gly His Gly Gly
395                 400                 405                 410

CAT CCC ATG CAG CAC AAC CCC AAT GGA CCC CCA CCC CCT TGG ATG CAG        1660
His Pro Met Gln His Asn Pro Asn Gly Pro Pro Pro Pro Trp Met Gln
                415                 420                 425

CCA CCA CCA CCA CCG ATG AAC CAG GGC CCC CAC CCT CCT GGG CAC CAT        1708
Pro Pro Pro Pro Pro Met Asn Gln Gly Pro His Pro Pro Gly His His
            430                 435                 440

GGC CCT CCT CCA ATG GAT CAG TAC CTG GGA AGT ACG CCT GTG GGC TCT        1756
Gly Pro Pro Pro Met Asp Gln Tyr Leu Gly Ser Thr Pro Val Gly Ser
        445                 450                 455

GGG GTC TAT CGC CTG CAT CAA GGA AAA GGT ATG ATG CCG CCA CCA CCT        1804
Gly Val Tyr Arg Leu His Gln Gly Lys Gly Met Met Pro Pro Pro Pro
    460                 465                 470

ATG GGC ATG ATG CCG CCG CCG CCG CCG CCT CCC AGT GGG CAG CCC CCA        1852
Met Gly Met Met Pro Pro Pro Pro Pro Pro Pro Ser Gly Gln Pro Pro
475                 480                 485                 490

CCC CCT CCC TCT GGT CCT CTT CCC CCA TGG CAA CAA CAG CAG CAG CAG        1900
Pro Pro Pro Ser Gly Pro Leu Pro Pro Trp Gln Gln Gln Gln Gln Gln
                495                 500                 505

CCT CCG CCA CCC CCT CCG CCC AGC AGC AGT ATG GCT TCC AGT ACC CCC        1948
Pro Pro Pro Pro Pro Pro Pro Ser Ser Ser Met Ala Ser Ser Thr Pro
            510                 515                 520

TTG CCA TGG CAG CAA AAT ACG ACG ACT ACC ACC ACG AGC GCT GGC ACA        1996
Leu Pro Trp Gln Gln Asn Thr Thr Thr Thr Thr Thr Ser Ala Gly Thr
        525                 530                 535

GGG TCC ATC CCG CCA TGG CAA CAG CAG CAG GCG GCT GCC GCA GCT TCT        2044
Gly Ser Ile Pro Pro Trp Gln Gln Gln Gln Ala Ala Ala Ala Ala Ser
    540                 545                 550

CCA GGA GCC CCT CAG ATG CAA GGC AAC CCC ACT ATG GTG CCC CTG CCC     2092
Pro Gly Ala Pro Gln Met Gln Gly Asn Pro Thr Met Val Pro Leu Pro
555                 560                 565                 570

CCC GGG GTC CAG CCG CCT CTG CCG CCT GGG GCC CCT CCC CCT CCG CCC     2140
Pro Gly Val Gln Pro Pro Leu Pro Pro Gly Ala Pro Pro Pro Pro Pro
                575                 580                 585

CGT AGC ATC GAG TGT CTT CTT TGT CTT CTT TCT CTC CTC ACC CAA CTC     2188
Arg Ser Ile Glu Cys Leu Leu Cys Leu Leu Ser Leu Leu Thr Gln Leu
            590                 595                 600

CCT TTG CCT CTC CCC AAA CCG GGC CGC CAG GAT CCC TCC CCG CGG CGG     2236
Pro Leu Pro Leu Pro Lys Pro Gly Arg Gln Asp Pro Ser Pro Arg Arg
        605                 610                 615

CGA TGG CCC GAG CCA TGA GAGTGAGGAC TTTCCGCGCC CATTGGTGAC            2284
Arg Trp Pro Glu Pro
    620

CCTTCCAGGC AGACAGCCTC AGCAACGCCC CTGGTGGACA GGATGGTTCG GCAAAGCAGC   2344

CTGAGTTATT TTTGTGGACG GAATCGGAAC ACGCTGGCTC CATATCGTGA AATTTTTATT   2404

AATTTTTTTC TTTTTCCTTT GTTACTTCTT TATCTTTTCC TTTCTTCAGA CTCCGTCCAA   2464

GGAGATGCTC TCCCCGGTCT TCTGCTGCAA TTTAGATTCC TTTGGGTTCT CTCCAGTTCT   2524

CCTTCCCTTA CCAAGGAGAG GGGAGCAAAT GGTTTTGGGC AAGGGCTTTG GCCATTCATG   2584

TCAAGCTGGT TGTGGGTTTT TCAAGGTGCC ATAGCCACCC CCAAATATGT TTGTTTAAAG   2644

CGTGGGGTTT TTTAATCTCT GCCACCCTTG TCAAGGGAGT CTTGTAAAGT TGCCGAGGGT   2704

AGGTTCATCT CCAGGTTTCG GGATTCCCAT CCGTCCTGGC GATCCTGCCA GCAGTGGGTG   2764

GGCAGCCTGA GCTCCCTCGG GCTCGCCTGC CAGCCTGGAG TTCTTCCTGT GCTCCTTGAT   2824

CACCTGAGCT GCCTCAGATT CCATTTGGTC CTCTCCTTCC TGGAAGGCTT CCTTTTATGT   2884

TTTGTTTTAA TCCCAAATGT CTGAATGTTT TGCAGTGTGT AGGGGTTTGA GCCCCTTGTT   2944

CATTCTCCTT CCTTTTTCCT CCCGCTTCCC TCTCCATGAA GTGATTCTGT TGACAATAAT   3004

GTATACTGCG CGTTCTCTTC ACTGGTTTAT CTGCAGAAAT TTCTCTGGGC TTTTTTCGGT   3064

GTTAGATTCA ACACTGCGCT AAAGCGGGGA TGTTCCATTG AATAAAAGAG CAGTGTGGTT   3124

TTCTGGGAAA AAAAAAAAAA AAAAAAAAAA AAAAAAAAAA AAAAAAAAAA AAAAAAAAAA   3184

AAAAAAAAAA AAAAAA                                                   3200

(2) INFORMATION FOR SEQ ID NO: 2:

    (i) SEQUENCE CHARACTERISTICS:
        (A) LENGTH: 623 amino acids
        (B) TYPE: amino acid
        (D) TOPOLOGY: linear

    (ii) MOLECULE TYPE: protein
    (xi) SEQUENCE DESCRIPTION: SEQ ID NO: 2:

Met Ala Thr Gly Ala Asn Ala Thr Pro Leu Asp Phe Pro Ser Lys Lys
1               5                   10                  15

Arg Lys Arg Ser Arg Trp Asn Gln Asp Thr Met Glu Gln Pro Thr Val
            20                  25                  30

Ile Pro Gly Met Pro Thr Val Ile Pro Pro Gly Leu Thr Arg Glu Gln
        35                  40                  45

Glu Arg Ala Tyr Ile Val Gln Leu Gln Ile Glu Asp Leu Thr Arg Lys
    50                  55                  60

Leu Arg Thr Gly Asp Leu Gly Ile Pro Pro Asn Pro Glu Asp Arg Ser
65                  70                  75                  80

Pro Ser Pro Glu Pro Ile Tyr Asn Ser Glu Gly Lys Arg Leu Asn Thr
                85                  90                  95

Arg Glu Phe Arg Thr Arg Lys Lys Leu Glu Glu Glu Arg His Asn Leu
            100                 105                 110

Ile Thr Glu Met Val Ala Leu Asn Pro Asp Phe Lys Pro Pro Ala Asp
        115                 120                 125

Tyr Lys Pro Pro Ala Thr Arg Val Ser Asp Lys Val Met Ile Pro Gln
    130                 135                 140

Asp Glu Tyr Pro Glu Ile Asn Phe Val Gly Leu Leu Ile Gly Pro Arg
145                 150                 155                 160

Gly Asn Thr Leu Lys Asn Ile Glu Lys Glu Cys Asn Ala Lys Ile Met
                165                 170                 175

Ile Arg Gly Lys Gly Ser Val Lys Glu Gly Lys Val Gly Arg Lys Asp
            180                 185                 190

Gly Gln Met Leu Pro Gly Glu Asp Glu Pro Leu His Ala Leu Val Thr
        195                 200                 205

Ala Asn Thr Met Glu Asn Val Lys Lys Ala Val Glu Gln Ile Arg Asn
    210                 215                 220

Ile Leu Lys Gln Gly Ile Glu Thr Pro Glu Asp Gln Asn Asp Leu Arg
225                 230                 235                 240

Lys Met Gln Leu Arg Glu Leu Ala Arg Leu Asn Gly Thr Leu Arg Glu
                245                 250                 255

Asp Asp Asn Arg Ile Leu Arg Pro Trp Gln Ser Ser Gly Thr Arg Ser
            260                 265                 270

Ile Thr Asn Thr Thr Val Cys Thr Lys Cys Gly Gly Ala Gly His Ile
        275                 280                 285

Ala Ser Asp Cys Lys Phe Gln Arg Pro Gly Asp Pro Gln Ser Ala Gln
    290                 295                 300

Asp Lys Ala Arg Met Asp Lys Glu Tyr Leu Ser Leu Met Ala Glu Leu
305                 310                 315                 320

Gly Glu Ala Pro Val Pro Ala Ser Val Gly Ser Thr Ser Gly Pro Ala
                325                 330                 335

Thr Thr Pro Leu Ala Ser Ala Pro Arg Pro Ala Ala Pro Ala Asn Asn
            340                 345                 350

Pro Pro Pro Pro Ser Leu Met Ser Thr Thr Gln Ser Arg Pro Pro Trp
        355                 360                 365

Met Asn Ser Gly Pro Ser Glu Ser Trp Pro Tyr His Gly Met His Gly
    370                 375                 380

Gly Gly Pro Gly Gly Pro Gly Gly Gly Pro His Ser Phe Pro His Pro
385                 390                 395                 400

Leu Pro Ser Leu Thr Gly Gly His Gly Gly His Pro Met Gln His Asn
                405                 410                 415

Pro Asn Gly Pro Pro Pro Pro Trp Met Gln Pro Pro Pro Pro Pro Met
            420                 425                 430

Asn Gln Gly Pro His Pro Pro Gly His His Gly Pro Pro Pro Met Asp
        435                 440                 445

Gln Tyr Leu Gly Ser Thr Pro Val Gly Ser Gly Val Tyr Arg Leu His
    450                 455                 460

Gln Gly Lys Gly Met Met Pro Pro Pro Pro Met Gly Met Met Pro Pro
465                 470                 475                 480

Pro Pro Pro Pro Pro Ser Gly Gln Pro Pro Pro Pro Pro Ser Gly Pro
                485                 490                 495

Leu Pro Pro Trp Gln Gln Gln Gln Gln Gln Pro Pro Pro Pro Pro Pro
            500                 505                 510

Pro Ser Ser Ser Met Ala Ser Ser Thr Pro Leu Pro Trp Gln Gln Asn
        515                 520                 525

Thr Thr Thr Thr Thr Thr Ser Ala Gly Thr Gly Ser Ile Pro Pro Trp
    530                 535                 540

Gln Gln Gln Gln Ala Ala Ala Ala Ala Ser Pro Gly Ala Pro Gln Met
545                 550                 555                 560

Gln Gly Asn Pro Thr Met Val Pro Leu Pro Pro Gly Val Gln Pro Pro
                565                 570                 575

Leu Pro Pro Gly Ala Pro Pro Pro Pro Pro Arg Ser Ile Glu Cys Leu
            580                 585                 590

Leu Cys Leu Leu Ser Leu Leu Thr Gln Leu Pro Leu Pro Leu Pro Lys
        595                 600                 605

Pro Gly Arg Gln Asp Pro Ser Pro Arg Arg Arg Trp Pro Glu Pro
    610                 615                 620

## Claims

1. A DNA comprising the full structure or a part of the DNA represented by SEQ ID NO:1 or a DNA essentially equal to the DNA comprising the full structure or a part of the DNA represented by SEQ ID NO:1.

2. A polypeptide comprising the full structure or a part of the polypeptide coded for by the DNA represented by SEQ ID NO:1 or a polypeptide essentially equal to the polypeptide comprising the full structure or a part of a polypeptide coded for by the DNA represented by SEQ ID NO:1.

3. A transformant transformed by a plasmid having, integrated therein, the full structure or a part of the DNA represented by SEQ ID NO:1 which can be expressed therein.

4. An antibody against the polypeptide as set forth in Claim 2 as an antigen.

5. A gene analysis method which comprises using, as a primer, a probe or a marker, a DNA comprising a part of the DNA represented by SEQ ID NO:1 and hybridizing the primer, the probe or the marker with a DNA to be tested.
